# FASCICULE DE BREVET EUROPEEN

(11) **EP 1 046 408 B1**
(45) Date de publication et mention de la délivrance du brevet: **14.09.2005**
(21) Numéro de dépôt: 00401096.3
(22) Date de dépôt: 19.04.2000
(51) Int. Cl.: A61N 1/375

(54) **Tête de connecteur à verrouillage rapide pour dispositif médical implantable actif**
Schnellsperrverbindungskopf für implantierbare medizinische Vorrichtung
Quick lock connector head for implantable medical device

(30) Priorité: 19.04.1999 FR 9904863
(43) Date de publication de la demande: 25.10.2000
(73) Titulaire: ELA MEDICAL (Société anonyme), 92541 Montrouge (FR)
(72) Inventeur: Ollivier, Jean-François, 91190 Villiers le Bade (FR)
(74) Mandataire: Dupuis-Latour, Dominique

(56) Documents cités:
- EP-A- 0 402 681
- US-A- 5 082 453
- US-A- 5 545 188

## Description

L'invention concerne les connecteurs pour dispositifs médicaux implantables actifs.

Elle sera principalement décrite dans le cas d'un stimulateur cardiaque, mais il ne s'agit là que d'un exemple de mise en oeuvre de l'invention, qui est applicable de façon beaucoup plus générale à une très grande variété de "dispositifs médicaux implantables actifs" tels que définis par la Directive 90/385/CE du 20 juin 1990 du Conseil des communautés européennes. Cette définition inclut, outre les stimulateurs cardiaques, les défibrillateurs et/ou cardioverteurs, les appareils neurologiques, les pompes de diffusion de substances médicales, les implants cochléaires, les capteurs biologiques implantés, etc.

Ces dispositifs comportent un boîtier, généralement désigné "générateur" ou "générateur d'impulsions", contenant l'électronique du dispositif et relié électriquement et mécaniquement à une ou plusieurs sondes.

Plus précisément, le boîtier du dispositif est constitué d'un corps contenant les divers circuits électroniques et la pile d'alimentation du dispositif, et d'une tête de connecteur solidaire du corps et pourvue d'un ou plusieurs logements susceptibles de recevoir la (les) sonde(s).

On pourra à cet effet se reporter à la norme française et européenne NF EN 50077 *"Connecteur à bas profil pour stimulateurs cardiaques implantables"*, qui définit un système de connexion normalisé dit "IS-1" permettant de garantir l'interchangeabilité des sondes et des générateurs d'impulsions produits par différents fabricants (l'invention n'est toutefois pas limitée au cas particulier des systèmes de connexion selon cette norme, ni même aux systèmes de connexion pour stimulateurs cardiaques).

Habituellement, le raccordement entre le (ou chaque) connecteur de sonde et la tête de connecteur du générateur est réalisé par une ou plusieurs vis, serrées par le chirurgien au moyen d'un outil *ad hoc* (tournevis muni éventuellement d'un limiteur de couple) au moment de l'implantation.

Ce raccordement par vis présente cependant plusieurs inconvénients.

En premier lieu, outre un outil spécifique pour sa mise en oeuvre, cette technique impose la présence de bouchons de passage étanche de cet outil pour éviter qu'après implantation l'extrémité de sonde insérée dans la tête de connecteur ne vienne en contact avec des fluides organiques. Cette exigence d'étanchéité au niveau des fentes de passage de l'outil lors de la connexion implique un surcoût et une augmentation de volume du générateur au niveau du connecteur.

Par ailleurs, ce système de raccordement n'est pas à l'abri d'un oubli de serrage de vis de la part du chirurgien, d'un serrage insuffisant ou d'un serrage trop violent endommageant le dispositif. Ce point est d'autant plus critique qu'il n'existe pas de moyen visuel de vérifier que le serrage a été correctement été opéré.

Ces inconvénients se trouvent particulièrement amplifiés dans le cas des implants utilisant un nombre important de sondes (trois, quatre, voire même plus), par exemple les dispositifs asservis nécessitant des capteurs physiologiques externes, ou encore les dispositifs de type "triple chambre", "quadruple chambre" ou encore "multisite" nécessitant l'implantation, et donc le raccordement, d'électrodes de détection et/ou de stimulation en plusieurs points du myocarde.

Un dispositif selon le préambule de la revendication 1 est connu du document US-A- 5 545 188.

Ainsi, un implant prévu pour recevoir quatre sondes comprend huit vis de serrage, ce qui implique sur le plan mécanique une structure volumineuse et complexe de tête de connecteur, en outre assez délicate à mettre en oeuvre (au moment de l'implantation, le chirurgien doit serrer huit vis, sans oubli ni serrage insuffisant ou excessif).

L'un des buts de l'invention est de remédier à ces divers inconvénients en proposant une structure de tête de connecteur qui soit à la fois simple à fabriquer et aisée à mettre en oeuvre par le praticien.

Plus précisément, le dispositif de connexion de l'invention, qui est destiné à un dispositif médical actif implantable comprenant un générateur et une tête de connecteur, est du type générique comportant une pluralité de logements axiaux parallèles aptes à recevoir des connecteurs de sonde respectifs, ainsi des moyens réversibles de retenue mécanique de chaque connecteur de sonde dans son logement.

Selon l'invention, les moyens réversibles de retenue comprennent un organe de manoeuvre commun mobile entre deux positions, verrouillée et déverrouillée, et des éléments escamotables actionnés simultanément par cet organe de manoeuvre commun, ces éléments escamotables étant aptes à faire saillie radialement à l'intérieur de chacun des logements respectifs en position verrouillée pour exercer une pression d'appui radiale sur chacun des connecteurs de sonde insérés dans leurs logements respectifs.

De préférence, le dispositif de connexion comprend en outre un connecteur de sonde comportant au moins un élément conducteur cylindrique annulaire et au moins une région électriquement isolante s'étendant de part et d'autre de cet élément conducteur cylindrique annulaire, et la pression d'appui radiale est exercée par les éléments escamotables sur ladite au moins une région électriquement isolante.

De préférence également, le dispositif de connexion comprend en outre un connecteur de sonde comportant un organe d'étanchéité de l'extrémité de sonde avec la tête de connecteur, et les éléments escamotables font saillie dans une région de l'extrémité de sonde située, dans le sens distal, au-delà de l'organe d'étanchéité.

Dans une forme de réalisation avantageuse, les logements femelles axiaux sont répartis régulièrement à une même distance d'un axe central de la tête de connecteur parallèle aux axes des logements, et les éléments escamotables sont portés par un barillet commun mobile en rotation autour de cet axe central, et avantageusement réalisé en un matériau élastiquement déformable, notamment de type silicone.

On va maintenant décrire un exemple de mise en oeuvre de l'invention en référence aux dessins annexés, sur lesquels les mêmes références numériques désignent des éléments identiques de l'exemple illustré.
La figure 1 est une vue en perspective arrachée d'une tête de connecteur selon l'invention, avec une extrémité de sonde avant insertion.
La figure 2 est une vue arrière de la tête de connecteur de la figure 1, en position déverrouillée permettant l'insertion de la tête de connecteur.
La figure 3 est homologue de la figure 1, après insertion de la tête de connecteur.
La figure 4 est homologue de la figure 2, en position verrouillée.

La référence 10 désigne de façon générale une tête de connecteur réalisée selon les enseignements de l'invention, formée en un matériau isolant rigide telle qu'un résine époxy (les têtes de connecteur connues étant habituellement réalisées en un matériau souple telle qu'une résine de silicone).

Cette tête 10 est pourvue d'une pluralité de logements 12. Dans l'exemple illustré, ces logements sont au nombre de quatre. Ils s'étendent en direction axiale parallèlement entre eux et sont régulièrement répartis autour d'un axe central D de la tête de connecteur, cet axe D étant parallèle aux axes des différents logements 12 et situé à même distance de ces derniers.

Chacun des logements 12 reçoit l'extrémité proximale d'une sonde (c'est-à-dire l'extrémité pourvue des moyens de connexion au générateur de l'implant), par exemple une sonde conforme au standard mécanique dimensionnel IS-1 mentionné plus haut (l'invention présente l'avantage d'être entièrement compatible avec ce standard IS-1, ce qui lui permet d'accepter sans modification toutes les sondes normalisées actuelles ou à venir).

L'extrémité de sonde 14 comporte, par exemple, deux éléments conducteurs cylindriques annulaires 16, 18 entourés par des régions isolantes 20, 22, typiquement en un matériau souple telle qu'une résine silicone. Les régions isolantes sont généralement pourvues de reliefs d'étanchéité annulaires 24, 26 destinés à coopérer avec les régions homologues de la paroi intérieure de la cavité 12 correspondante.

Chacune des cavités 12 contient une ou deux bornes (selon que la sonde correspondante est monopolaire ou, comme illustré, bipolaire) assurant la liaison électrique entre l'élément conducteur 16 ou 18 et une entrée/sortie du circuit du générateur de l'implant. (l'invention ne concernant pas la liaison électrique de la sonde au connecteur et étant indépendante de la technique choisie pour assurer cette liaison, celle-ci ne sera pas décrite plus avant).

L'invention concerne la solidarisation mécanique de la tête de connecteur aux différentes extrémités de sonde une fois celles-ci toutes insérées dans leurs logements 12 respectifs.

Dans le mode de réalisation préférentiel illustré (qui n'est pas limitatif), cette liaison mécanique de verrouillage est assurée par un barillet central 28, mobile en rotation autour de l'axe D et comportant une pluralité de régions en creux 30, en nombre égal à celui des logements 12 et séparées par un nombre égal d'éléments en saillie 32 répartis périphériquement de façon régulière autour de l'axe D. Le barillet 28 pivote autour d'une pièce fixe 34 pourvue d'un palier recevant le barillet 28, ce dernier étant solidaire d'une tige 36 susceptible d'être entraînée en rotation par un organe d'entraînement 38, pourvu par exemple d'une fente 40 destinée à recevoir la lame d'un tournevis. En direction axiale, l'organe 38 est situé à l'extrémité de la tête de connecteur qui est opposée à celle sur lesquelles débouchent les logements 12 recevant les connecteurs de sonde 14.

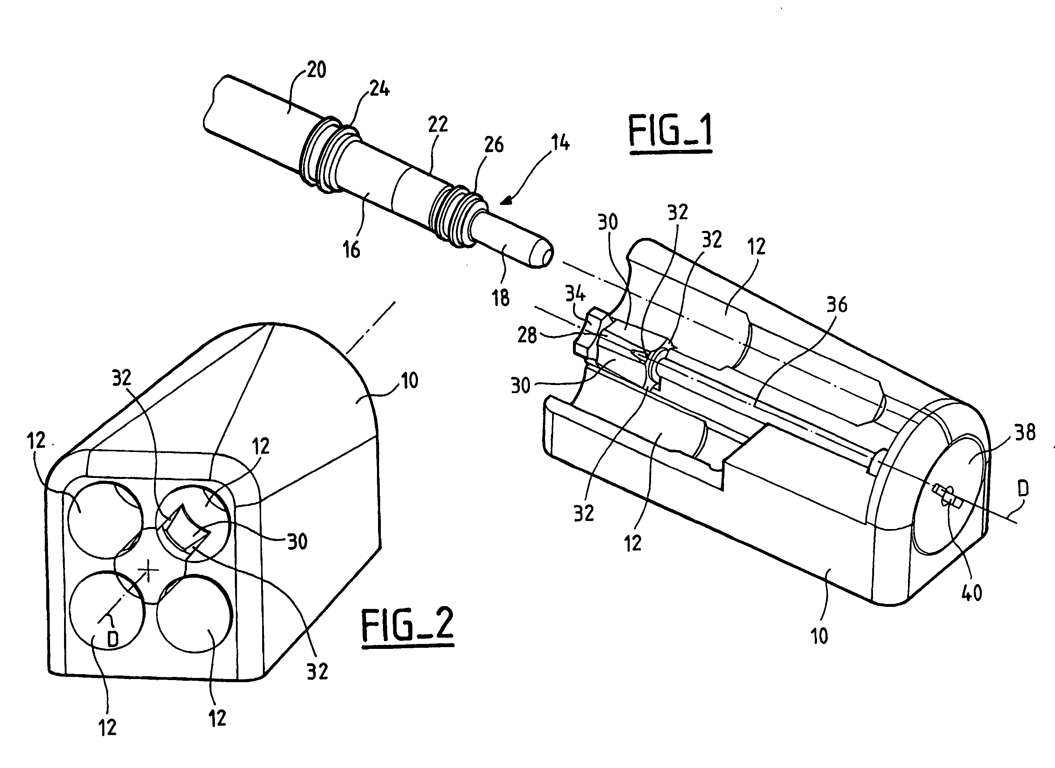

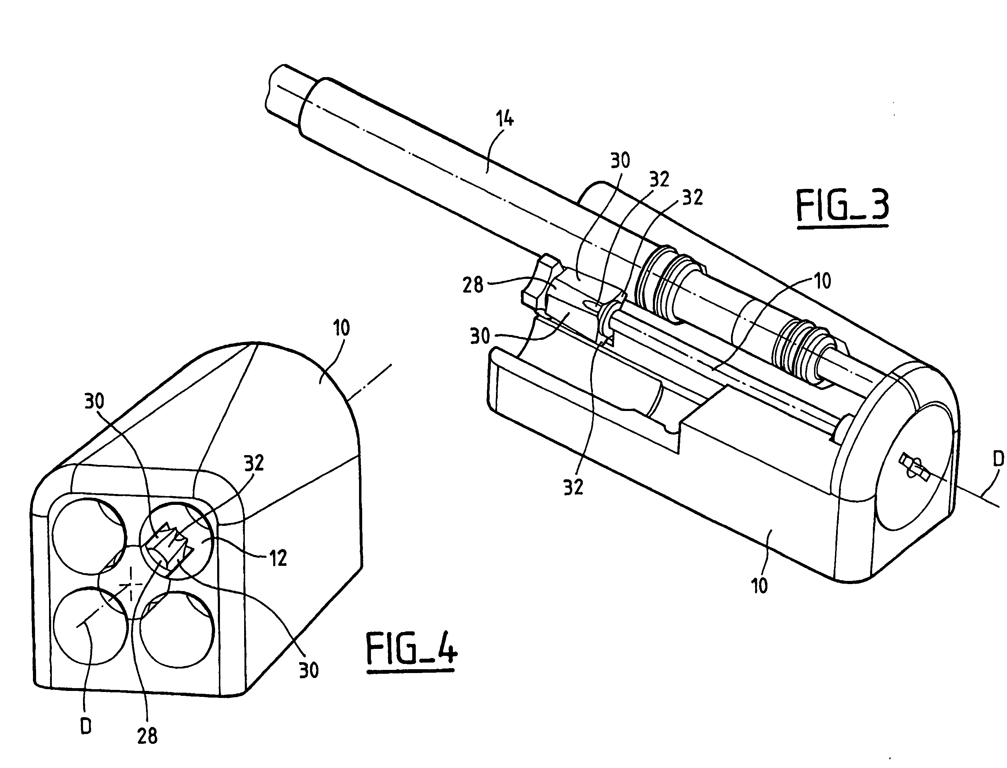

Le barillet 28 est susceptible de prendre deux positions, illustrées respectivement sur les figures 2 et 4, le passage d'une position à l'autre s'effectuant par un rotation d'1/8^{e} de tour dans l'exemple illustré, c'est-à-dire avec quatre logements 12 et donc quatre éléments en saillie 32.

Dans la première position (position déverrouillée), illustrée figure 2, les régions en creux 30, qui sont de préférence des régions présentant une surface cylindrique concave de même courbure que celles de la région correspondante du logement 12, viennent affleurer la paroi du logement 12 ; extérieurement, chacun de ces logements 12 se présente donc avec une forme cylindrique, sans obstacle, permettant donc l'insertion aisée du connecteur de sonde 14 à fond dans son logement.

Une fois les quatre connecteurs de sonde insérés dans leurs logements respectifs, il suffit de faire pivoter d'un 1/8^{e} de tour l'organe de manoeuvre 38, ce qui a pour effet de faire passer le barillet 28 à la position illustrée figure 4.

Dans cette seconde position (position verrouillée), les éléments 32 font saillie à l'intérieur des logements 12. Du fait de leur forme allongée, ces éléments en saillie 32 viennent appuyer le long d'une génératrice du connecteur de sonde dans la région de la gaine isolante silicone 20.

Le silicone - du connecteur et/ou du barillet - étant un matériau incompressible (déformation à volume constant), il suffit de dimensionner les éléments en saillie 32, c'est-à-dire de choisir la surface de la région en appui contre la gaine silicone et la dimension de la saillie, de manière à obtenir la force de rétention voulue, typiquement la valeur prescrite par la norme IS-1 qui définit précisément à la fois la force de rétention à exercer sur un connecteur de sonde et la zone sur laquelle cette force peut être exercée.

Le mode de réalisation que l'on vient de décrire n'est pas limitatif.

Notamment, le système de barillet rotatif peut être remplacé par exemple par un dispositif dans lequel les éléments en saillie peuvent être escamotés ou déployés à l'intérieur du logement 12 par un système combinant une vis (entraînée par l'organe de manoeuvre 38) avec un mécanisme à écartement.

Dans ce cas, le mouvement des éléments en saillie est uniquement un déplacement radial dans la cavité 12, sans rotation autour d'un axe central.

## Revendications

1. Un dispositif de connexion pour dispositif médical actif implantable, notamment stimulateur cardiaque, défibrillateur et/ou cardioverteur, comprenant un générateur et une tête de connecteur (10),
ce dispositif de connexion comportant une pluralité de logements axiaux parallèles (12) aptes à recevoir des connecteurs de sonde (14) respectifs, ainsi que des moyens réversibles de retenue mécanique de chaque connecteur de sonde dans son logement,
et étant **caractérisé en ce que** les moyens réversibles de retenue comprennent un organe de manoeuvre commun (36, 38, 40) mobile entre deux positions, verrouillée et déverrouillée, et des éléments escamotables (32) actionnés simultanément par cet organe de manoeuvre commun, ces éléments escamotables étant aptes à faire saillie radialement à l'intérieur de chacun des logements respectifs en position verrouillée pour exercer une pression d'appui radiale sur chacun des connecteurs de sonde insérés dans leurs logements respectifs.

2. Le dispositif de connexion de la revendication 1, comprenant en outre un connecteur de sonde comportant au moins un élément conducteur cylindrique annulaire (16, 18) et au moins une région électriquement isolante (20 ; 22) s'étendant de part et d'autre de cet élément conducteur cylindrique annulaire, et dans lequel ladite pression d'appui radiale est exercée par les éléments escamotables sur ladite au moins une (20) région électriquement isolante.

3. Le dispositif de connexion de la revendication 1, comprenant en outre un connecteur de sonde comportant un organe (24) d'étanchéité de l'extrémité de sonde avec la tête de connecteur, et dans lequel les éléments escamotables font saillie dans une région (20) de l'extrémité de sonde située, dans le sens distal, au-delà de l'organe d'étanchéité.

4. Le dispositif de connexion de la revendication 1, dans lequel les logements femelles axiaux sont répartis régulièrement à une même distance d'un axe central (D) de la tête de connecteur parallèle aux axes des logements, et les éléments escamotables (32) sont portés par un barillet commun (28) mobile en rotation autour de cet axe central.

5. Le dispositif de connexion de la revendication 4, dans lequel le barillet (28) est en un matériau élastiquement déformable, notamment de type silicone.

## Patentansprüche

1. Verbindungsvonrichtung für eine medizinische aktive implantierbare Vorrichtung, insbesondere Herzschrittmacher, Defibrillator und/oder Kardioverter, aufweisend einen Generator und einen Verbinderkopf (10),
wobei diese Verbindungsvorrichtung eine Mehrzahl von axialen, parallelen Aufnahmen (12) aufweist, die fähig sind, jeweils Sondenverbinder (14) aufzunehmen, sowie mechanische, reversible Haltemittel jedes Sondenverbinders in seiner Aufnahme,
und **dadurch gekennzeichnet, dass** die reversiblen Haltemittel ein gemeinsames Betätigungsorgan (36, 38, 40) aufweisen, welches zwischen zwei Stellungen, einer verriegelten und einer entriegelten, beweglich ist, und einziehbare Elemente (32), welche gleichzeitig durch dieses gemeinsame Betätigungsorgan betätigt werden, wobei diese einziehbaren Elemente fähig sind, radial ins Innere jeder der jeweiligen Aufnahmen in verriegelter Stellung vorzuragen, um einen radialen Haltedruck auf jeden der Sondenverbinder auszuüben, die in ihren jeweiligen Aufnahmen eingeführt sind.

2. Verbindungsvorrichtung nach Anspruch 1, des Weiteren aufweisend einen Sondenverbinder, welcher mindestens ein zylindrisches, ringförmiges Leiterelement (16, 18) aufweist und mindestens einen clektrische isolierenden Bereich (20, 22), der sich beiderseits dieses zylindrischen, ringförmigen Leiterelements erstreckt, und bei welcher der radiale Haltedruck durch die einziehbaren Elemente auf den mindestens einen (20) elektrisch isolierenden Bereich ausgeübt wird.

3. Verbindungsvorrichtung nach Anspruch 1, des Weiteren aufweisend einen Sondenverbinder, welcher ein Dichtigkeitsbauteil (24) des Endes der Sonde mit dem Kopf des Verbinders aufweist, und bei welcher die einziehbaren Elemente in einen Bereich (20) des Endes der Sonde vorragen, welches in distaler Richtung auf der anderen Seite des Dichtigkeitsbauteils angeordnet ist.

4. Verbindungsvorrichtung nach Anspruch 1, in welcher die weiblichen, axialen Aufnahmen gleichmäßig in einem gleichen Abstand zu einer mittigen Achse (D) des Verbinderkopfes parallel zu den Achsen der Aufnahmen verteilt sind und die einziehbaren Elemente (32) durch einen gemeinsamen Zylinder (28), der in Drehung um diese mittige Achse beweglich ist, getragen werden.

5. Verbindungsvorrichtung nach Anspruch 4, bei welcher der Zylinder (28) aus einem elastisch verformbaren Material, insbesondere vom Typ Silikon, ist.

## Claims

1. A connection system for an active implantable medical device, in particular a cardiac pacemaker, defibrillator and/or cardioverter, including a generator and a connector head (10),
said connection system comprising a plurality of parallel axial cavities adapted to receive respective lead connectors (14), and reversible means for mechanically tightening each lead connector within its cavity,
and being **characterised in that** the reversible tightening means includes a common actuation member (36, 38, 40) movable between two positions, locked and unlocked, and a retractable elements (32) actuated simultaneously by said common actuation member, said retractable elements being adapted to radially project inside each respective cavity in said locked position for exerting a radial contact pressure on each of the lead connectors inserted into their respective cavities.

2. The connection system of claim 1, further including a lead connector comprising at least one annular cylindrical conductive element (16, 18) and at least one electrically insulating area (20 ; 22) extending across said annular cylindrical conductive element, and wherein said radial contact pressure is exerted by the retractable elements on said at least one (20) electrically insulated area.

3. The connection system of claim 1, further including a lead connector comprising at least one member (24) for sealing the lead tip with the connector head, and wherein the retractable elements project into an area (20) of the lead tip distally located beyond the sealing member.

4. The connection system of claim 1, wherein the axial female cavities are regularly distributed at the same distance about a central axis (D) of the connector head parallel to the axes of the cavities, and the retractable elements are supported by a common barrel (28) pivotally movable around said central axis.

5. The connection system of claim 4, wherein the barrel (28) is of an elastically deformable material, in particular a silicone type material.
